**Europäisches Patentamt**

**European Patent Office**

**Office européen des brevets**

(19)

(11) Veröffentlichungsnummer: **0 278 374**

**A2**

## (12) EUROPÄISCHE PATENTANMELDUNG

(21) Anmeldenummer: **88101519.2**

(22) Anmeldetag: **03.02.88**

(51) Int. Cl.⁴: **G01N 13/00**

(30) Priorität: **06.02.87 DE 3703621**

(43) Veröffentlichungstag der Anmeldung:
**17.08.88 Patentblatt 88/33**

(84) Benannte Vertragsstaaten:
**CH DE FR GB IT LI**

(71) Anmelder: **PHARMATEST APPARATEBAU GMBH**
**Dieselstrasse 10**
**D-6452 Hainburg(DE)**

(72) Erfinder: **Eppelmann, Heinz**
**Johannes-Calvin-Strasse 15**
**D-6507 Ingelheim(DE)**
Erfinder: **Fähler, Franz**
**Birkenwaldstrasse 35**
**D-6053 Obertshausen(DE)**

(74) Vertreter: **Linser, Heinz**
**Patentanwälte Heinz Linser Dipl. Ing.**
**Eckhardt Eyer Robert-Bosch-Strasse 12a**
**Postfach 10 22 10**
**D-6072 Dreieich(DE)**

(54) **Vorrichtung zur Bestimmung der Wirkstoff-Freigabe von pharmazeutischen Produkten.**

(57) Die Erfindung betrifft eine Vorrichtung zur Bestimmung der Wirkstoff-Freigabe von lösbaren pharmazeutischen Produkten in oralen Darreichungsformen, insbesondere von Tabletten, Pillen, Kapseln und Tropfen, umfassend eine in einem Gestell angeordnete Vielzahl von Gefäßen mit jeweils auf-und niederbewegbaren sowie zentral antreibbaren Rühren und Einrichtungen zur Befüllung und Entleerung der Gefäße mit entsprechenden Proben und Flüssigkeiten. Hierbei ist einem Gestell (1) eine zentrale Steuer-, Antriebs-und Beschickungseinheit sowie Pumpeinrichtung (40) zugeordnet, der Boden eines jeden Gefäßes (2) ist mit einem von der zentralen Steuereinheit automatisch betätigbaren Ventil (12) zum Öffnen und Schließen versehen, jedem Ventil schließt sich eine Rohr-oder Schlauchverbindung (13) an, welche mit der zentralen Pump-oder Unterdruckpumpeinrichtung (40) verbunden ist und der Rührer (7) weist einen Rührkörper (15) auf, welcher mit einem als Hohlrohr ausgebildeten Rührschaft verbunden ist. Auf oder in dem Rührkörper (15) sind mit dem Hohlrohr verbundene und zum äußeren Rand der Rührfläche verlaufende Kanäle mit freien Kanalausgängen angeordnet.

EP 0 278 374 A2

Fig. 2

## Vorrichtung zur Bestimmung der Wirkstoff-Freigabe von pharmazeutischen Produkten

Die Erfindung betrifft eine Vorrichtung zur Bestimmung der Wirkstoff-Freigabe von lösbaren pharmazeutischen Produkten in oralen Darreichungsformen, insbesondere von Tabletten, Pillen, Kapseln und Tropfen, umfassend eine in einem Gestell angeordnet Vielzahl von Gefäßen mit jeweils auf-und niederbewegbaren sowie zentral antreibbaren Rühren und Einrichtungen zur Befüllung und Entleerung der Gefäße mit entsprechenden Flüssigkeiten.

Die Wirkstoff-Freigabe-Prüfung bedeutet die Ermittlung des Wirkstoffgehaltes aus einem Medikament unter kontrollierten und konstanten Bedingungen der Prüftemperatur, der Rührergeschwindigkeit, der Analysenintervalle und weiterer Parameter. Eine solche Untersuchung wird in nationalen (DAB: Deutsches Arzneibuch) und auch internationalen Bestimmungen, beispielsweise in der sogennanten USP (US-Pharmakopoe) und BP (British Pharmakopoe) be-und vorgeschrieben.

Eine solche Prüfung ist neben der Qualitätskontrolle ein wichtiges Beurteilungskriterium während der Entwicklung dieser Präparate.

Die gültigen und o.g. Prüfvorschriften beschreiben hierbei ausführlich die Apparatur hinsichtlich ihres Aussehens, ihrer Größe, des Materials, der Rührerkonstruktion und der einzuhaltenden Toleranzen der wesentlichsten Apparate-Bestandteile.

Mindestens sechs Prüflinge sollen gleichzeitig unter konstanten Bedingungen untersucht werden, wobei die Anzahl der Probenentnahmen von eins bis über zwanzig reichen kann.

Die Bestimmung der Wirkstoff-Freigabe aus festen und lösbaren pharmazeutischen Produkten ist eine besonders arbeitsintensive und zeitaufwendige Arbeit in pharma-analytischen Laboratorien.

Für diese Bestimmung sind bereits Vorrichtungen bekannt, welche den eingangs genannten Vorschriften genügen, beispielsweise die Wirkstoff-Freisetzungsapparatur PTW 12 S der Firma PHARMATEST GMBH, D-6452 Hainburg.

Eine solche Vorrichtung besteht in der Hauptsache aus einer Reihe von Gefäßen, die jeweils mit einem Rührer ausgerüstet sind, welche zentral angetrieben werden. Die Gefäße besitzen im wesentlichen eine zylindrische Form und einen halbkugelförmigen Boden. Die Beschickung, Probeentnahme und Reinigung erfolgen von der oben offenen Seite des Zylinders aus von Hand.

Hierdurch ist der Betriebsablauf nicht optimal und führt zu vermeidbaren Störungen. So erfolgt die Reinigung der Gefäße hintereinander, wodurch, bereits eine erhebliche Zeit benötigt wird.

Der Erfindung liegt die Aufgabe zugrunde, die erforderlichen Betriebsabläufe, insbesondere die Beschickung und Reinigung der Gefäße sowie auch die Probeentnahme vollständig zu automatisieren, wodurch die Leistungsfähigkeit der Apparate erheblich verbessert werden kann.

Die Lösung dieser Aufgabe erfolgt gemäß der Erfindung dadurch, daß bei der eingangs genannten Vorrichtung

1.1 dem Gestell eine zentrale Steuer-, Antriebs-und Beschickungseinheit sowie Pumpeinrichtung zugeordnet ist;

1.2 der Boden eines jeden Gefäßes mit einem von der zentralen Steuereinheit automatisch betätigbaren Ventil zum Öffnen und Schließen versehen ist,

1.3 jedem Ventil sich eine Rohr-oder Schlauchverbindung anschließt, welche mit einer zentralen Pump-oder Unterdruckpumpeinrichtung verbunden ist und

1.4 der Rührer einen Rührkörper aufweist, welcher mit einem als Hohlrohr ausgebildeten Rührschaft verbunden ist und auf oder in dem Rührkörper mit dem Hohlrohr verbundene und zum äußeren Rand der Rührfläche verlaufende Kanäle mit freien Kanalausgängen angeordnet sind.

In Weiterbildung der Erfindung weist das Gestell ein eine Hub-und Senkbewegungen durchführbares Oberteil auf, durch as die Rührschäfte der Rührkörper jeweils mittels eines Drehlagers hindurchgeführt sind, so daß eine Drehbewegung der Rührschäfte ermöglicht wird und eine Mitnahme bei einer Hub-und Senkbewegung erfolgt. Hierbei ist in vorteilhafter Weise in dem Oberteil des Gestells ein durch einen Motor angetriebener Riemenantrieb angeordnet und die Rührschäfte der Rührkörper weisen jeweils mindestens eine Reimenscheibe zum Eingriff für die Antriebsbänder des Riemenantriebe und Druchführung der Drehbewegung des Rührers auf.

Nach der Erfindung ist mit dem Boden eines Gefäßes ein Ventilteil verschraubt, das ein Anschlußstück aufweist, welches beispielsweise als Olive ausgebildet sein kann und eine koaxial angeordnete, in ihrem unteren Teil mit einer oder mehreren Öffnungen versehene enthält, in der ein Stopfen, der unter dem Druck einer Feder in die Öffnung im Boden des Gefäßes gepreßt wird, verschiebbar angeordnet ist.

Der Rührkörper ist in vorteilhafter Weise als Blattrührer ausgebildet und weist an seinem oberen Ende einen Anschlußstutzen zur Zuführung von Flüssigkeit oder Luft auf, wobei der Hohlraum im Rührerschaft mit den Kanälen im Rührkörper strömungsmäßig verbunden ist. Durch die Zuführung von Flüssigkeit, vorzugsweise Wasser durch den Anschlußstutzen und damit durch die sich anschließenden Kanäle, kann nunmehr das Gefäß vollständig gereinigt werden, da der blattrührer während der Zuführung eines Fluids gleichzeitig sowohl eine Dreh-als auch eine Auf- und Abbewegung durchführen kann.

Nach Ablauf der Reinigungsflüssigkeit aus dem Gefäß durch das automatisch betätigbare Ventil im Boden des Gefäßes kann Luft durch den Anschlußstutzen in das Gefäß geblasen werden, wodurch eine Trocknung erreicht wird. Da die Reinigungs-und Trocknungsarbeiten für sämtliche Gefäße einer Vorrichtung gleichzeitig durchgeführt werden können, ist die gesamte Vorrichtung in äußerst kurzer Zeit wieder einsatzbereit.

In vorteilhafter Weiterbildung der Erfindung ist dem Gestell mindestens ein Tablettenmagazin zugeordnet. Es kann hierbei · auch jedem Gefäß ein eigenes Tablettenmagazin zugeordnet werden, so daß bei dieser Ausführungsform eine besondere Zuführungsvorrichtung entfällt. In allen anderen Fällen sind entsprechende über die Länge des Gestells sich erstreckende, verschiebbar ausgebildete Einwurfschienen oder auch eine einzelne Einwurfschiene zur automatischen Versorgung eines jeden Gefäßes mit Meßproben zugeordnet.

Die Wirkstoff-Friegabegefäße werden nach der Erfindung somit in der Weise modifiziert und angeordnet, daß ein automatisches Zuführen der zu untersuchenden Proben und ein Entleeren des Auflösemediums mit abnschließendem Spül-und Trochnungsvorgang nach Abschluß eines Wirkstoff-Freisetzungs-Versuchs ermöglicht wird. Sämtlich Arbeitsgänge erfolgen nach der Erfindung derartig, daß alle Gefäße in einem Gestell gleichzeitig behandelt werden, so daß bereits hierdurch eine erhebliche Zeit eingespart wird. Damit läßt sich eine solche Vorrichtung auch mittels prozessorgesteuerter Rechner vollständig steuern und überwachen, wobei damit auch eine Protokollführung gegeben ist.

Die Erfindung wird anhand der Figuren näher erläutert. Hierbei zeigen

FIGUR 1 eine Vorderansicht eines gesamten Systems mit Probensammler, Meßvorrichtung nach der Erfindung, Pumpvorrichtung und Steuerkomputer;

FIGUR 2a eine Vorderansicht der Meß-und Pumpvorrichtung nach Figur 1 in einem geringfügig vergrößertem Maßstab;

FIGUR 2B eine Seitenansicht der Meßvorrichtung nach Figur 2a;

FIGUR 2c eine Draufsicht auf die Meßvorrichtung nach Figur 2a;

FIGUR 3 eine Draufsicht auf die Meßvorrichtung, welche mit einer Meßprobenzuführeinrichtung ausgerüstet ist;

FIGUR 4 eine Draufsicht auf eine Meßvorrichtung im Längsschnitt durch das Oberteil mit Antrieb;

FIGUR 5 eine im Vergleich zu den Figuren 1 bis 4 vergrößerte Darstellung des unteren Teils eines Gefäßes mit einem angebauten automatischen Ventil;

FIGUR 6 einen Längsschnitt durch einen Rührkörper gleichgroßer Darstellung zur Figur 5 ;

FIGUR 7 eine Draufsicht auf den linken Teil des Blattrührers nach Figur 6;

FIGUR 8 eine Draufsicht auf den rechten Teil des Blattrührers nach Figur 6;

FIGUR 9 der obere Teil eines Rührschaftes.

In den Figuren sind gleiche Elemente mit gleichen Bezugszeichen dargestellt.

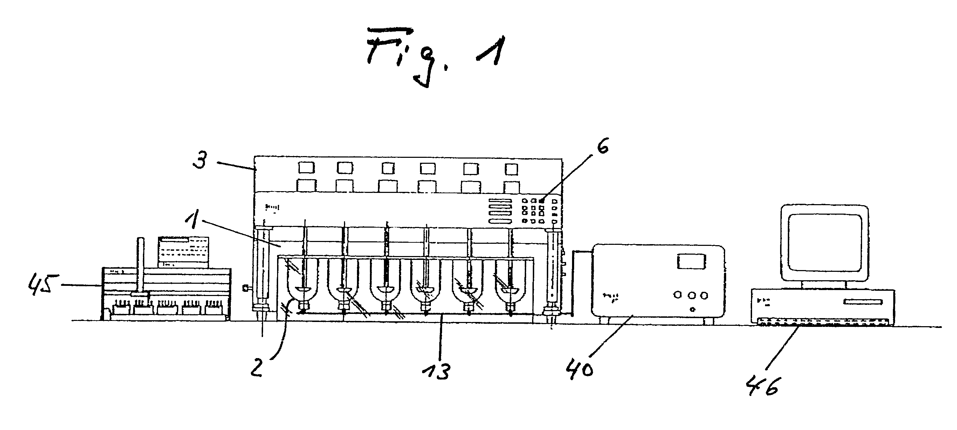

Die Figur 1 gibt einen Überblick über das gesamte Meßsystem. Zentrales Gerät ist dabei die Meßvorricht mit ihrem Gestell 1, welches die Gefäße 2 für die Lösung der Meßprogen enthält.

Eine Drucktasteneinheit 6 dient zur Bedienung der Meßvorrichtung. Im Oberteil 3 befindet sich der Antrieb der in den Gefäßen 2 befindlichen Rührer, auf den später noch näher eingegangen wird. Eine Sammelleitung 13 verbindet die einzelnen Gefäße 2 mit der Pumpeneinrichtung 40. Mit 45 ist ein Probensammler oder Sprecktralphotometer bezeichnet, mit dem die Analyse bzw. Auswertung der Ergebnisse aus der Meßvorrichtung durchgeführt wird. Er ist nicht Gegenstand der Erfindung. Entsprechendes gilt für den Rechner 46, welcher zur Steuerung des gesamten Systems dient.

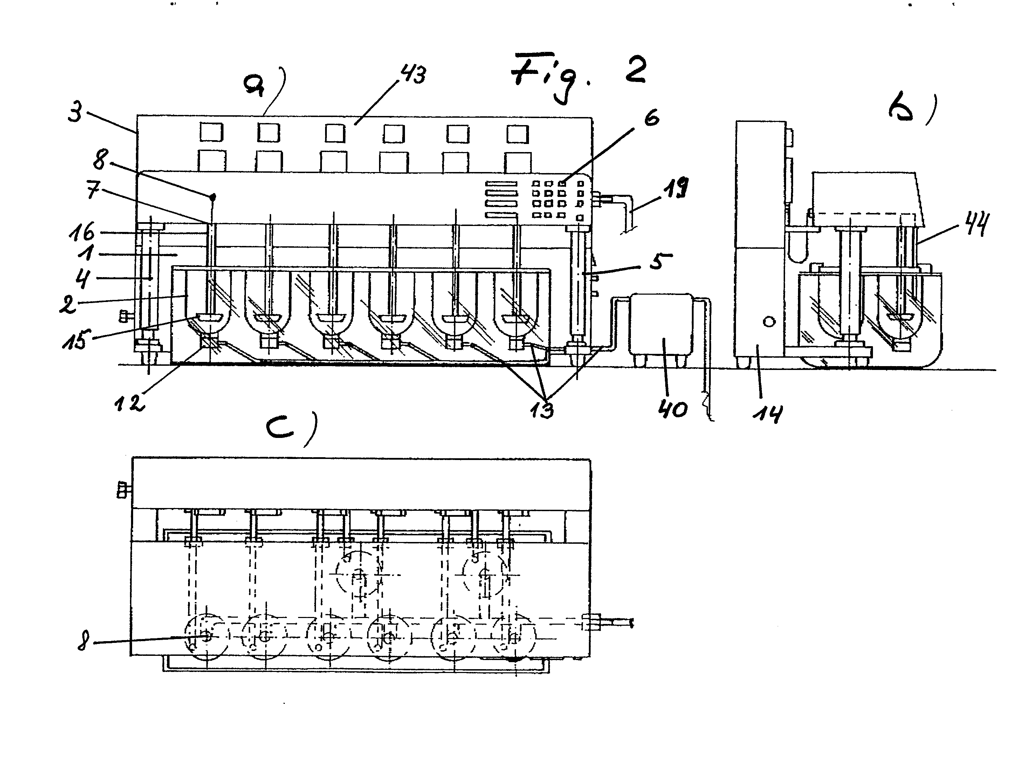

Die Figuren 2a, 2b und 2c zeigen die Meßvorrichtung in Vorderansicht, Seitenansicht und Draufsicht in geringfügig vergrößerter Darstellung in Vergleich zur Figur 1. Das Gestell 1 enthält acht Gefäße 2. Das Oberteil 3 ist mit Hilfe von Hubkolben 4 und 5 auf-und abbewegbar. Es enthält eine Drucktasteneinheit 6 auf seiner Frontseite zur Betätigung einer automatischen Steuerung.

Die in den Gefäßen 2 befindlichen Rpührkörper 15 der Rührer 7 sind an der oberen Enden ihrer Rührschäfte 16 im Oberteil 3 des Steuergehäuses 43 drehbar gelagert, jedoch derart, daß sie bei einer Hubbewegung des Oberteils 3 ebenfalls auf-und abbewegt werden.

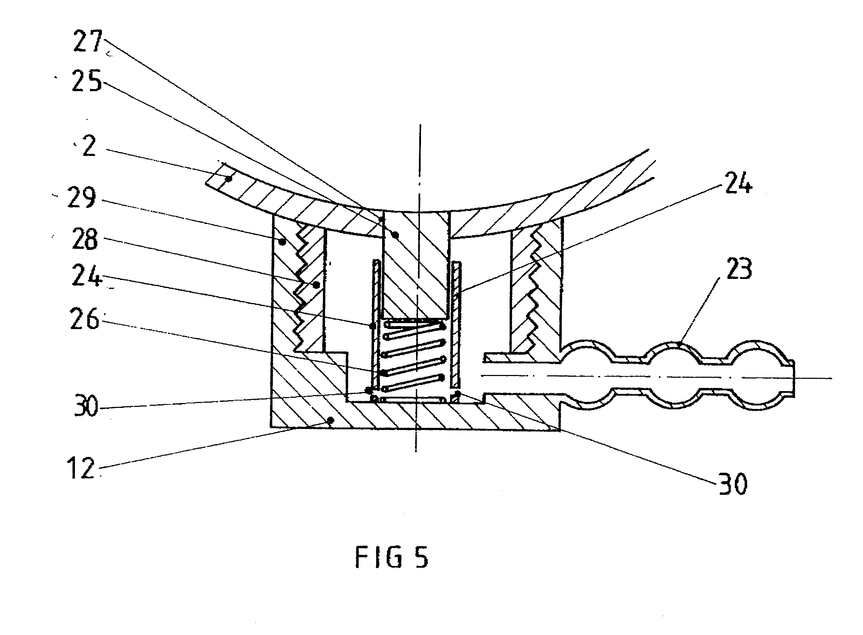

Jedes Gefäß 2 ist in seinem unteren Bodenteil mit einem Ventil 12 versehen, welches automatisch betätigt werden kann und noch später anhand der Figur 5 näher beschrieben wird. Jedes Ventil 12 ist über eine Schlauchleitung 13 mit einer Pumpenvorrichtung 40 verbunden, welche beispielsweise auch als Saugpumpe ausgebildet sein kann.

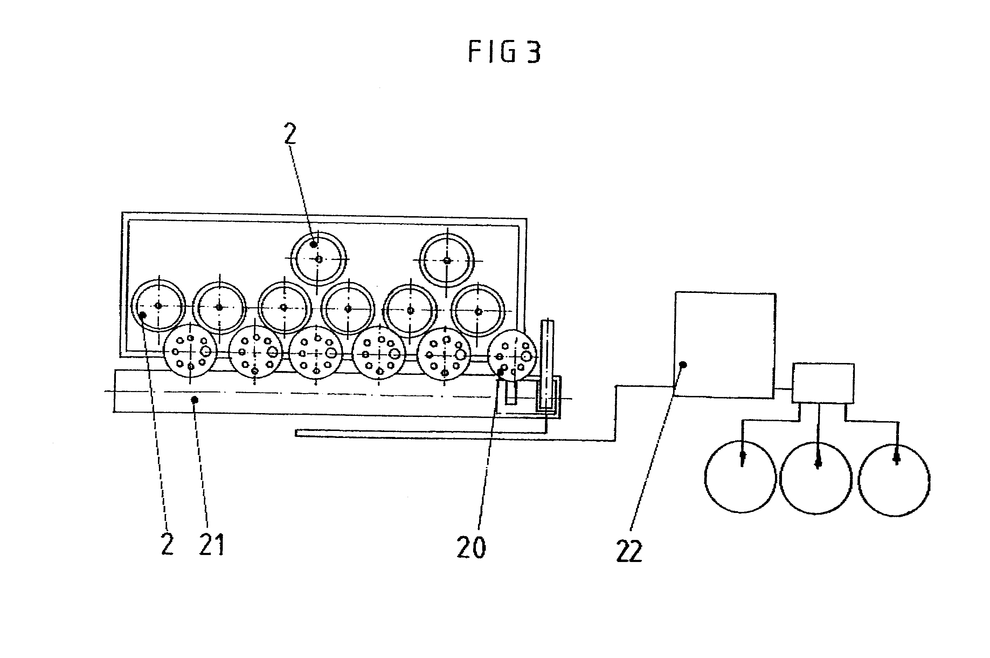

Die Figur 3 zeigt ferner Tabelettenmagazine 20 für die automatische Beschickung der Gefäße 2. Das Magazin 20 übergibt die zu den Gefäßen 2 zu transportierenden Tabletten einer Einwurfschiene 21, welche entlang dem Gestell 1 derartig verfahrbar ausgebildet ist, daß die Meßproben in die jeweiligen Gefäße fallen können.

Mittels entsprechender und freizugebender Löcher in der Einwurfschiene 21 werden die Gefäße 2 in gleicher Linie gleichzeitig beschickt. Die Steuerung 22 steuert den gesamten Ablauf der Beschickung, Entleerung und Reinigung der Gefäße 2 und kann vom Rechner 46 (s. Figur 1) übernommen werden.

Während des Arbeitsprozesses ist es erforderlich, die Rührschäfte 16 der gesamten Rührer 7 in Drehung zu versetzen, während für den Reinigungsprozeß eine Hub-und Senkbewegung durchgeführt werden muß.

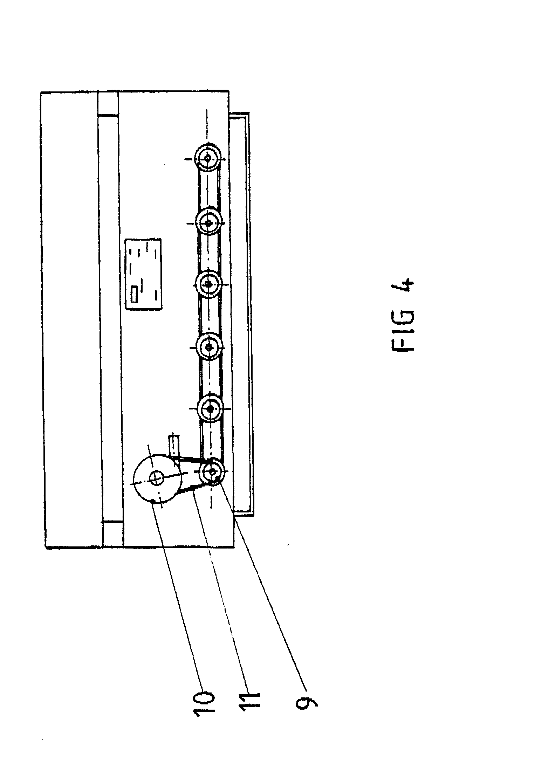

Hierzu wird auf die Figur 4 verwiesen, welche einen horizontalen Schnitt in Draufsicht durch das Oberteil 3 des Steuergehäuses einer ähnlich ausgebildeten Meßvorrichtung zeigt, die jedoch lediglich sechs Gefäße 2 enthält, wobei alle oben beschriebenen Mittel und Funktionen gleichartig sind.

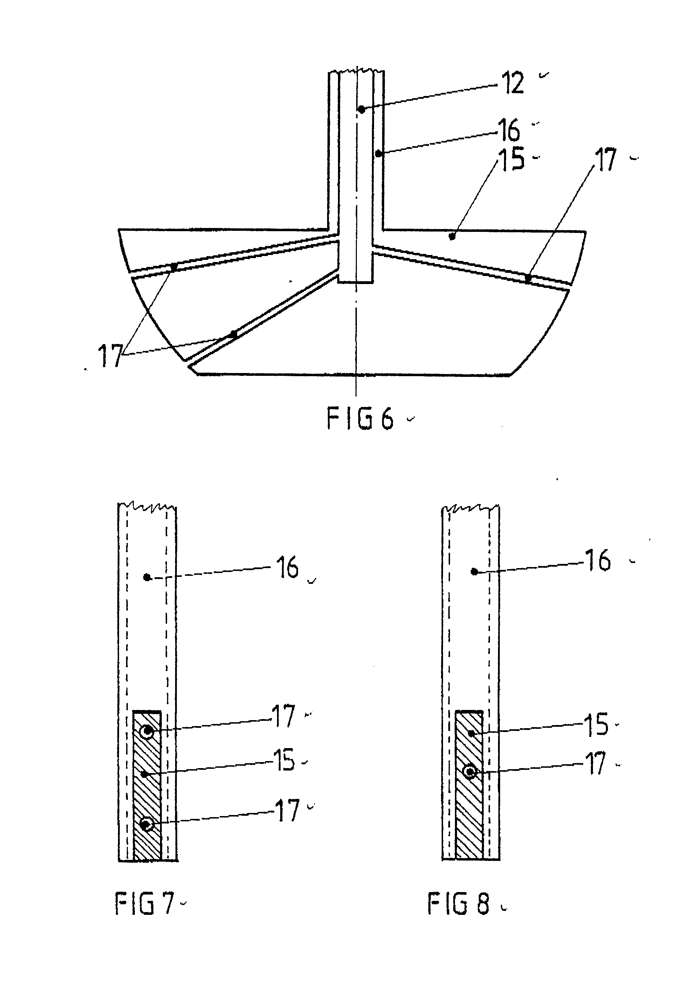

Hierbei sind die oberen Enden der Rührschäfte 16 jeweils mit einem Antriebsrad 9 zur Durchführung einer Drehbewegung versehen, welches jeweils von einem mit dem Motor 10 direkt oder indirekt verbundenen Antriebsband 11 angetrieben wird. Da der Antriebmechanismus im Oberteil 3 angeordnet ist, nimmt er an der Hubbewegung teil und ermöglicht gleichzeitig, d.h. auch während der Hubbewegung eine Drehbewegung der Rührschäfte 16 und damit der Rührkörper 15 in den Gefäßen 2. Der Rührkörper 15, dessen spezielle Ausbildung später anhand der Figur 6 beschrieben wird, kann damit entlang der inneren Wände der Gefäße 2 bewegt werden und Reinigungsarbeiten vollautomatisch durchführen.

Die Figur 5 zeigt einen vergrößerten Ausschnitt des Bodens eines Gefäßes 2 mit einem angebauten Ventil 12 zum automatischen Öffnen und Schließen des Gefäßes 2.

Mit dem Gefäßboden ist ein Außengewinde 28 fest verbunden. Auf das Gewinde ist ein Ventilteil 29 fest und dicht aufgeschraubt, das mit einer Olive 23 als Anschlußstück versehen ist.

Anstelle einer Olive 23 kann auch ein zylindrisches Rohrstück verwendet werden. In dem Ventilteil 29 ist eine mit seitlichen Öffnungen 30 versehene Führungshülse 24 koaxial angeordnet. Die Führungshülse 24 umschließt teilweise den frei verschiebbaren Stopfen 25, der von einer Feder 26 bei Fehlen einer äußeren Einwirkung in die Öffnung 27 des Gefäßbodens gepreßt wird.

Anstelle des druckgesteuerten Ventils 12 kann beispielsweise auch ein nicht näher dargestelltes Magnetventil verwendet werden.

Die Funktionsweise des Ventils ergibt sich aus dem dargestellen Aufbau.

Nach Beendigung der Wirkstoff-Freisetzungs-Bestimmung wird an der Olive 23 durch die dort angeschlossene Pumpeneinrichtung 40 ein Unterdruck erzeugt. Die Pumpe kann beispielsweise als Wasserstrahlpumpe ausgebildet sein. Durch den erzeugten Unterdruck wird der Stopfen 25 gegen den Druck der Feder 26 nach unten gezogen, so daß die Öffnung 27 im Boden des Gefäßes 2 geoffnet wird. Das Medium im Gefäß 2 wird durch die Öffnung 27, Rohranschluß 23, Pumpeneingang 14 und Pumpe 40 in ein nicht näher dargestelltes Abfallgefäß gesaugt. Nach Beendigung des Vorgangs wird die Wasserstrahlpumpe 40 abgeschaltet. Die Feder 26 preßt nun den Stopfen 25 wieder in die Öffnung 27, so daß das Gefäß 2 geschlossen ist. Anschließend erfolgt die gründliche Reinigung des Gefäßes, welche nach jedem Prozeß erforderlich ist, wie im folgenden beschrieben wird.

Die Figur 6 zeigt den unteren Teil des Rührers 7. Der Rührkörper 15 besteht hierbei aus einem flächenhaft ausgebildeten Körper, einem sogenannten Blattrührer, welcher von im wesentlichen radial verlaufenden Kanälen 17 durchzogen ist, die mit dem Hohlrohr des Rührschaftes 16 strömungsmäßig verbunden sind. Wird dem Rührschaft 16 unter regulierter Einführung Reinigungsflüssigkeit über den Anschlußstutzen 19 zugeführt und der Rührer 7 mit seinem Rührkörper 15 in Drehung versetzt, so erzeugt bereits die auf die in den Kanälen 17 befindliche Flüssigkeit wirkende Zentrifugalkraft ein Ausspritzen der Flüssigkeit gegen die inneren Wandungen des Gefäßes 2. Wird nunmehr gleichzeitig eine Hubbewegung des Oberteils 3 durchgeführt, so können dadurch alle inneren Teile der Wandungen von der ausgespritzen Reinigungsflüssigkeit erreicht werden. Mit veränderbarem Druck wird ein ausreichend scharfer Flüssigkeitsstrahl erreicht, so daß eine vollständige Reinigung garantiert werden kann.

Während der Zufuhr der Reinigungsflüssigkeit bleibt der Stopfen 25 in Schließstellung, sodaß sich das Gefäß 2 füllt und dadurch auch der Rührkörper 15 selbst gesäubert wird. Anschließend wird die Spülflüssigkeit durch die Öffnung 27 im Gefäßboden entfernt und der Reinigungsvorgang kann, falls es erforderlich ist, wiederholt werden. Zum Schluß werden das Gefäß und der Rührer mit Druckluft, welche durch den Rührer geblasen wird, getrocknet.

Nach der Reinigung kann ein Freisetzungsmedium in das Gefäß gefüllt werden und nach Erreichen der Temperaturkonstanz das Testobjekt, z.B. eine Tablette, zur automatischen Bestimmung der Wirkstoff-Freisetzung eingeworfen werden.

Figur 7 zeigt die Draufsicht von unten des linken Teils des Rührkörpers 15 nach Figur 6 und läßt die beiden Öffnungen der Kanäle 17 erkennen.

Figur 8 zeigt die Draufsicht von unten des rechten Teils des Rührkörpers 15 mit der einen Öffnung des Kanals 17 nach Figur 6. Wie diesbezüglich aus der Figur 6 zu erkennen ist, sind die Öffnungen der Kanäle in ihrer Wirkung auf die gegenüberstehende Wandung des Gefäßes 2 über die Höhe des Rührkörpers 15 gleichmäßig verteilt, ohne daß der mechanische Aufbau des Rührkörpers 15 ungünstig beeinflußt wird.

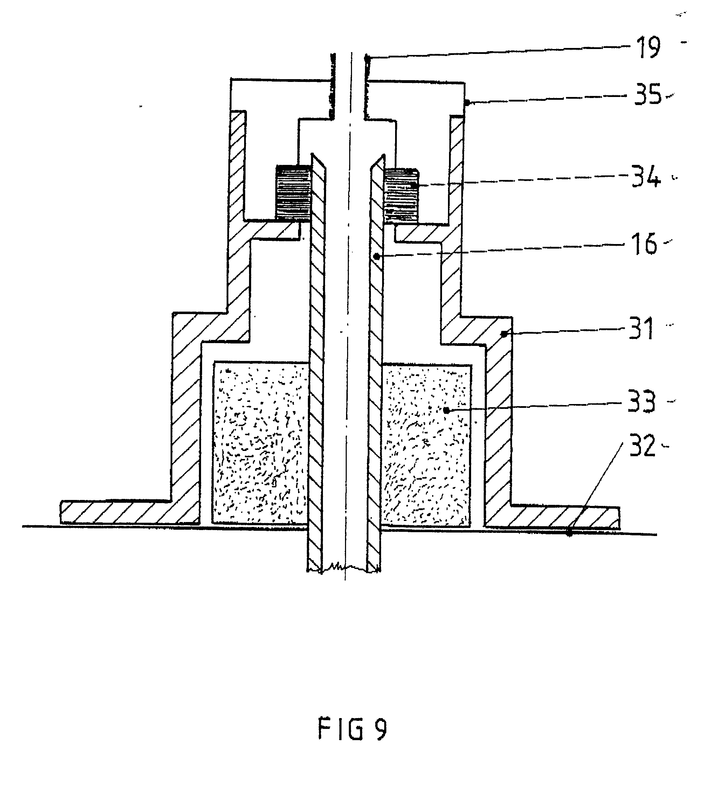

In Figur 9 sind der obere Teil des Rührerschafts 16 und seine Umgebung in einem senkrechten Schnitt entlang der Mittelachse dargestellt. Das Gehäuse 31 befindet sich oberhalb des Antriebsteils 32, umschließt das obere Ende des Rührerschafts 16, die Kontermutter 33 und die Simmerringe 34, die von der Verschraubung 35 fixiert werden. Die Verschraubung 35 ist mit dem Anschlußstück 19 ausgestattet, durch das die Zufuhr von Flüssigkeit, vorzugsweise Wasser oder Luft in den Rührkörper 15 erfolgt.

Erst die Kombination der verbesserten Wirkstoff-Freisetzungsgefäße mit den verbesserten Rührern und mit der bekannten Freisetzungsapparatur sowie einer verbesserten Transportvorrichtung für die Beschickung der Gefäße mit Untersuchungsgut und die Zusammenwirkung dieser Elemente ermöglicht die Automatisierung der Meßverfahren und der weiteren Prozeduren. Die Probeentnahme erfolgt über Schlauchpumpen, wobei die Lösungen dem Probensammler 45 durch den Probenzug 44 (s. hierzu Figur 1 und Figur 2b) zugeführt werden.

Für die Automatisierung werden zusätzlich elektrische bzw. magnetische Ventile zur Steuerung von Druckluft, Wasserzufuhr und Vakuumerzeugung, z.B. über Wasserstrahlpumpen, verwendet. Sämtliche Funktionen der modifizierten Freisetzungsapparatur werden von dem Mikrocomputer 46 in an sich bekannter Weise gesteuert, so daß sich die manuelle Bearbeitungszeit auf die Festlegung der Parameter vor Beginn der Versuchsreihe und auf die Beschickung der Transportschiene mit Untersuchungsgut beschränkt.

Durch die Tatsache, daß alle Arbeitsvorgänge bezüglich der einzelnen Gefäße 2 nach der Erfindung gleichzeitig erfolgen, wird ein erheblicher Rationalisierungseffekt und ein wirtschaftlicher Einsatz des gesamten Meßsystems erreicht.

## Ansprüche

1. Vorrichtung zur Bestimmung der Wirkstoff-Freigabe von lösbaren pharmazeutischen Produkten in oralen Darreichungsformen, insbesondere von Tabletten, Pillen, Kapseln und Tropfen, umfassend eine in einem Gestell angeordnete Vielzahl von Gefäßen mit jeweils aufund niederbewegbaren sowie zentral antreibbaren Rührern und Einrichtungen zur Befüllung und Entleerung der Gefäße mit entsprechenden Proben und Flüssigkeiten, dadurch gekennzeichnet, daß

1.1 dem Gestell (1) eine zentrale Steuer-, Antriebs-und Beschickungseinheit sowie Pumpeinrichtung (40) zugeordnet ist;

1.2 der Boden eines jeden Gefäßes (2) mit einem von der zentralen Steuereinheit automatisch betätigbaren Ventil (12) zum Öffnen und Schließen versehen ist,

1.3 jedem Ventil sich eine Rohr-oder Schlauchverbindung (13) anschließt, welche mit der zentralen Pump-oder Unterdruckpumpeinrichtung (40) verbunden ist und

1.4 der Rührer (7) einen Rührkörper (15) aufweist, welcher mit einem als Hohlrohr ausgebildeten Rührschaft (16) verbunden ist und auf oder in dem Rührkörper (15) mit dem Hohlrohr verbundene und zum äußeren Rand der Rührfläche verlaufende Kanäle (17) mit freien Kanalausgängen angeordnet sind.

2. Vorrichtung nach Anspruch 1, dadurch gekennzeichnet, daß das Gestell (1) ein eine Hub-und Senkbewegungen durchführbares Oberteil (3) aufweist, durch das die Rührschäfte (16) der Rührkörper (15) jeweils mittels eines Drehlagers (8) geführt sind, so daß eine Drehbewegung der Rührschäfte (16) ermöglicht wird und eine Mitnahme bei einer Hub-und Senkbewegung erfolgt.

3. Vorrichtung nach Anspruch 2, dadurch gekennzeichnet, daß in dem Oberteil (3) des Gestells (1) ein durch einen Motor (10) angetriebener Riemenantrieb angeordnet ist und die Rührschäfte (16) der Rührkörper (15) jeweils mindestens eine Reimenscheibe (9) zum Eingriff für die Antriebsbänder (11) des Reimenantriebe und Durchführung der Drehbewegung des Rührkörpers (15) aufweisen.

4. Vorrichtung nach Anspruch 1 oder einem der voranstehenden Ansprüche, dadurch gekennzeichnet, daß mit dem Boden eines Gefäßes (2) ein Ventil (12) befestigt ist, das ein Anschlußstück aufweist und eine koaxial angeordnete, in ihrem unteren Teil mit einer oder mehreren Öffnungen versehen Hülse (24) enthält, in der ein Stopfen (25), der unter dem Druck einer Feder (26) in die Öffnung (27) im Boden des Gefäßes (2) gepreßt wird, verschiebbar angeordnet ist.

5. Vorrichtung nach Anspruch 1 oder der voranstehende Ansprüche, dadurch gekennzeichnet, daß der Rührkörper (15) als Blattrührer ausgebildet ist und an seinem oberen Ende einen Anschlußstutzen (19) zur Zuführung von Flüssigkeit oder Luft aufweist, wobei der Hohlraum in Rührerschaft mit den Kanälen (17) im Rührkörper (15) strömungsmäßig verbunden ist.

6. Vorrichtung nach Anspruch 1 oder oder einem der voranstehenden Ansprüche, dadurch gekennzeichnet, daß dem Gestell (1) mindestens ein Tablettenmagazin (20) zugeordnet ist.

7. Vorrichtung nach Anspruch 1 oder oder einem der voranstehenden Ansprüche, dadurch gekennzeichnet, daß jedem Gefäß (2) ein Tablettenmagazin (20) zugeordnet ist.

8. Vorrichtung nach Anspruch 6, dadurch gekennzeichnet, daß dem Gestell (1) ein Tablettenmagazin (20) mit einer über die Länge des Gestells (1) sich erstreckenden, verschiebbar ausgebildeten Einwurfschiene (21) zur automatischen Versorgung eines jeden Gefäßes (2) mit Meßproben zugeordnet ist.

Fig. 1

Fig. 2

a)

b)

c)

0 278 374

FIG 3

2

2  21

20

22

0 278 374

FIG 4

0 278 374

27

25

2

29

28

24

26

30

12

24

23

30

0 278 374

FIG 5

FIG 6

FIG 7

FIG 8

19

35

34

16

31

33

32

FIG 9